# EUROPEAN PATENT SPECIFICATION

(11) **EP 2 406 365 B1**
(45) Date of publication and mention of the grant of the patent: **04.09.2013**
(21) Application number: 10713119.5
(22) Date of filing: 08.03.2010
(51) Int. Cl.: C12N 1/14, C12N 3/00, C12N 1/32, C12G 1/02

(54) **COMPOSITIONS SUITABLE FOR BOTRYTIZATION**
ZUR BOTRYTISIERUNG GEEIGNETE ZUSAMMENSETZUNGEN
COMPOSITIONS ADAPTÉES POUR LA BOTRYTISATION

(30) Priority: 09.03.2009 EP 09425093
(43) Date of publication of application: 18.01.2012
(73) Proprietor: Biotecnologie BT S.r.l., 06050 Todi (PG) (IT)
(72) Inventor: FRESCHI, Giorgio, I-29100 Piacenza (IT); CORANELLI, Simona, I-06030 Giano dell'Umbria (Perugia) (IT); VANNINI, Andrea, I-1030 Canepina (Viterbo) (IT); VUONO, Giorgio, I-87060 San Giorgio Albanese (Cosenza) (IT); CHILOSI, Gabriele, I-00100 Roma (IT)
(74) Representative: Gerli, Paolo
(86) International application number: PCT/EP2010/001418
(87) International publication number: WO 2010/102768

(56) References cited:
- EP-A1- 1 862 075
- WO-A1-99/05310
- WO-A2-2006/068481
- DE-B- 1 158 310
- US-A- 4 393 083
- DELCAN J ET AL: "Storage of Botrytis cinerea using different methods" JOURNAL OF PLANT PATHOLOGY, vol. 84, no. 1, March 2002 (2002-03), pages 3-9, XP009124874 ISSN: 1125-4653
- RAPOSO R ET AL: "Application of an automated quantitative method to determine fungicide resistance in Botrytis cinerea" PLANT DISEASE, vol. 79, no. 3, 1995, pages 294-296, XP009124873 ISSN: 0191-2917
- GINDRO KATIA ET AL: "Effects of long-term storage at different temperatures on conidia of Botrytis cinerea Pers.: Fr" FEMS MICROBIOLOGY LETTERS, vol. 204, no. 1, 16 October 2001 (2001-10-16), pages 101-104, XP002552789 ISSN: 0378-1097
- DATABASE WPI Week 197228 Thomson Scientific, London, GB; AN 1972-44972T XP002601583 & SU 317 699 A1 (DAGESTAN AGRICULTURAL INS) 1971
- NELSON K E: "Use of Botrytis Cinerea for the Production of Sweet Table Wines" AMERICAN JOURNAL OF ENOLOGY AND VITICULTURE, AMERICAN SOCIETY FOR ENOLOGY AND VITICULTURE, US, vol. 7, no. 4, 1 January 1956 (1956-01-01) , pages 131-136, XP009124848 ISSN: 0002-9254

## Description

### Field of the invention

The present invention relates to new compositions particularly suitable for performing grape dehydration, for increasing grape sugar content and for modifying grape aromatic composition, which allows obtaining valuable wines with high alcoholic content, characteristic flavour and distinctive taste.

### State of the art

Grapes are naturally subjected to infection with a number of environmental fungal microorganisms, most of them (e.g. *Aspergillus spp, Rhizopus nigricans, Penicillum spp*.) being detrimental to grape and wine quality. Some fungi however, in particular *Botrytis cinerea,* have since long been exploited in the production of sweet, liqueur-type wines. In fact under particular environmental conditions these fungi dehydrate the infected grapes to a controlled degree, increasing their sugar content and modifying their flavour balance; the thus infected grapes, after undergoing to the normal fermentation process, result in the production of highly valuable aromatic wines, such as Sauternes (France), Tokaj (Hungary), Trockenauslese (Germany), etc.

Workable conditions suitable for controlled *Botrytis* growth are in general quite restricted: they generally require a moist weather during the initial infection phase, followed by drier weather allowing evaporation from the infected fruit; heat should then remain moderate, as higher temperatures disturb. That can have a negative influence on the *Botrytis* metabolism for example to favour the growth of unwanted competitive microorganisms (Nelson K.E., Am.J.Enol., 1956, 7-131-6) For the above mentioned reasons, the biological production of good quality sweet wines, is limited to very narrow areas as compared to the generally known wine-producing regions.

Scientific studies on controlled botrytization date back to the beginning of the last century. P.Carles (Bullettin de l'Association des.Chemistes de Sucre et de Distilleries de France et de Colonies., 1910, 27, 777-781) reports that grapes infected with *Botrytis cinerea* allow obtaining sweet liqueur-type wines, mainly due to the enhanced sugar content of the infected grapes.

Dittrich H.H. et al. (Vitis, 1974, 13(1), 36-49) performed a comparative analysis of must from healthy or *Botrytis*-infected grapes: the latter showed initially a lower contents of sugar and tartaric acid, as well as higher amounts of malic, gluconic, pyruvic and chetoglutaric acids, all being useful to fungal growth; the fungal metabolism then caused a pH enhancement and favoured, upon fermentation, the formation of sweet and delicate wines.

Dugo P. et al. (Riv.Ital.Sci.Aliment., 1994, 23(2), 215-225) describes the influence of *Botrytis cinerea* upon the volatile components and aromatic alcohols responsible for the organoleptic characteristic of Cabernet Sauvignon and Sauvignon Blanc.

Studies showing correlations between certain environmental parameters and the growth degree of *Botrytis cinerea* are also available (e.g. Thomas C.S. et al., Phytopatology, 1988, 78(3), 260-265).

The above studies, while providing a valid insight into the mechanisms of botrytization, fail to provide botrytization methods alternative to the traditional one. Such alternative methods would be highly desirable e.g. in the aim of increasing the yield and/or reduce the complex environmental requirements for obtaining botrytized wines.

Some authors have performed botrytization by means of *Botrytis cinerea* preparations.

For example, Nelson K.E. (op. cit.) infected grapes with an extemporaneous spore suspension: after 1 week incubation at 20°C in controlled humidity conditions, a sweet wine with a typical flavour was obtained. The patent applications SU 317699 discloses a method of producing dessert wines, whereby a grape vinasse is contacted with a pure culture of *Botrytis cinerea*, followed by fermentation. US patent 4,393,083 describes a method to produce botrytized grape must whereby a grape must is added with moist mycelia of *Botrytis cinerea* obtained from a conventional Botrytis-containing culture, and with 3'5' adenylic acid or derivatives thereof. JP58094381 describes the preparation of a fruit wine by adding a wet mold of *Botrytis cinerea* to suitable fruits or fruit juices.

The above methods have disadvantages in that they remain heavily reliant on the quality of the *Botrytis* inoculus, which must be cultured and maintained viable until the moment of contacting the grapes; unfortunately, the quality of the cultured media varies over time with respect to fungal concentration, viability, and germination potency; furthermore the used *Botrytis* sampling methods do not allow collecting uniform and homogeneous amounts of fungi: consequently the infection is not homogeneously inoculated and is hardly reproducible over time. In these conditions, the environmental controlling factors remain predominant to ensure proper evolution of the growth in the desired controlled degree, whereas slight modifications thereof may result in a significant worsening of wine quality. In addition all the above study merely use fresh preparations of *Botrytis* inocula; from the industrial point of view it would be convenient to avoid obtain extemporaneous inocula from lively *Botrytis* cultures, but rather maintain a stock of non-growing inoculation precursors capable to germinate only when administered to the substrate; unfortunately, conidia are very labile to environmental conditions, therefore any attempts to temporarily inhibit their germination potency, tends to evolve in a drastic permanent reduction thereof.

Delcan J. et al., J.Plant Pathology 84(1), March 2002, p. 3-9 describes the storage of Botrytis cinerea spores in glycerol (20%) at -20°C. The patent application DE 1 158 310 describes a foam with added Botrytis spores to enhance the noble rot in grapes.

### Summary of the invention

The present inventors have obtained a new product capable of providing a highly viable, constant and reproducible source of conidia of *Botrytis cinerea,* suitable for the botrytization of grapes. The product of the invention is a conide-containing preparation, the germinability of which can be temporarily inhibited, thus allowing prolonged storage without premature fungal growth. The inhibition is not permanent, i.e. it does not substantially interfere with the inherent germination capability of Botrytis *cinerea* conidia. The product of the invention comprises conidia of *Botrytis cinerea,* at least one C3-6 polyol, and at least one monobasic alkaline salt. Suitable preformulates to obtain said product are also part of the invention. Upon use, a simple activation procedure starts the conidia germination process, yielding a highly activated homogeneous inoculum in the liquid phase. The administration of a measured volume of this product e.g. via spraying, allows dispersing a high and reproducible amount of fungi over the entire surface of target substrate, thus promoting a uniform and controlled growth. The invention includes methods to perform botrytization of grapes, involving the use of the product herein described; methods to produce a botryitized wine, involving the use of the said product, and the wine thus obtained.

### Detailed description

The aqueous medium used to prepare the present compositions is preferably chosen among demineralised water, distilled water, or physiological solution; the medium is preferably sterile, to prevent contamination by foreign microorganisms.

The Botrytis conidia concentration is preferably comprised between 1 X 10⁶ and 6 X 10⁶ conidia / ml, more preferably between 2,2 X 10⁶ and 4,4 X 10⁶ conidia /ml, e.g. 3.1X 10⁶ conidia/ml. This concentration can however be varied beyond the above intervals depending on the circumstances of administration.

The number of conidia present in the composition (and/or in the fungal raw materials used to prepare it), can be assessed by known methods, eg. by means of a Thoma chamber as described in the experimental part.

The conidia of *Botrytis cinerea* can be obtained by standard means from any cultures of these fungi. Convenient growing media, growing and harvesting conditions for *Botrytis cinerea* are known in the art (CMI.1974. Description of Pathogenic Fungi and Bacteria, No.43 1, Botrytis cinerea, Commonwealth Mycological Institute, England; Faretra F. and Grindle M. (1992) Genetic studies of Botryotinia fuckeliana/Botrytis cinerea. In: Verhoeff Malathrakis Williamson (Eds) Recent Advances in Botrytis research (pp-7-17) Pudoc Scientific Publishers, Wageningen, NL) and are exemplified in the experimental part. A general non limiting procedure includes inoculating and culturing *Botrytis cinerea* into a standard culture medium (e.g. Potato dextrose agar or Sabouraud dextrose agar). The culture is incubated at a temperature ranging from 15 to 27°C, at relative humidity above 80%, for a time comprised between 15 and 20 days. After this period, a conidia-rich mycelia layer of 0.5-3 mm thickness develops upon the surface of the culture medium. The fungal raw material is collected as a powdered material by removing the surface of the culture media.

It is not essential for the present composition to contain purified conidia, i.e. the fungal material present in the composition may well include other parts of *Botrytis* mycelia and parts of *Botrytis* culture medium, with no significant effect on the efficacy of the composition.

In the compositions of the invention the C3-6 polyol is preferably chosen among glycerol, mannitol, xylitol, sorbitol and mixtures thereof; glycerol is particularly preferred. The polyol may be present in amounts preferably comprised between 50 and 300 g/l, more preferably between 90 and 120 g/l. When more polyols are used, the above ranges are meant to refer to the total weight of present polyols. This component has particular utility in preserving the conidia vitality, particularly during storage.

The monobasic alkaline salt is preferably a sodium or potassium salt; more preferred are monobasic sodium phosphate or monobasic potassium phosphate. A combination of more of said monobasic alkaline salts can also be used for the purpose of the invention.

The monobasic alkaline salt is used in amounts suitable to provide a concentration comprised between 0.01 and 0.1 M, more preferably between 0.03 and 0.08 M, e.g. 0.07, and a pH comprised between 4 and 6, preferably between 4.4 and 5.7. When more monobasic alkaline salts are used, the above molarity ranges are meant to refer to the total moles of said salts.

The monobasic alkaline salt is essential in the administration phase to promote germinability, although it is not essential for the composition during storage; therefore, if desired, this component can be omitted from the original composition and added separately thereto prior to use. Accordingly, the present invention also includes a kit comprising aqueous preformulate compositions comprising conidia of *Botrytis cinerea* and at least one C3-6 polyol, said preformulate being meant for mixing, prior to administration, with one or more monobasic alkaline salts.

Additional ingredients, though not mandatory, may also be present in the above compositions/preformulates: examples of these ingredients are preservatives, surface active agents, etc., all of them being quali- and quantitatively compatible with the specific substrate to be subjected to botrytization.

The compositions of the invention, or the corresponding preformulates, can be stocked for a considerable amount of time, preferably more than 1 year at temperatures comprised between -15° and -25°C, or more than one month at temperatures between 3° and 5°C, with substantially complete inhibition of fungal growth, yet allowing a very high level of germination upon administration.

After storage and prior to use, the composition is turned into liquid phase by bringing it at temperatures above 0°C: if no germination is desired during this phase, the solution is suitably maintained between 3° and 5°C; total inhibition of germination is however no longer mandatory at this stage, therefore the solution may be brought to higher temperatures e.g. 5-10°C or more conveniently to ambient temperature, and then administered to the fruit. The temperature increase may be obtained by known means, e.g. via a thermostated bath. If a preformulate composition was used, this procedure is completed by adding the monobasic alkaline salt until reaching a pH between 4 and 6.

As highlighted in the experimental part, the activated composition shows a remarkable germination potency (constantly falling within the 60-100% range, allowing for an efficient contamination of the treated substrate.

Although the invention is widely described with reference to the advantageous mode of administrating after storage, it is also possible for it to be administered immediately after preparation, without prior storage: this mode is also part of the claimed invention.

Depending on circumstances, the composition of the invention may be diluted prior to use with further amounts of an aqueous medium as defined above, until reaching a volume sufficient to contact the entire surface of the substrate to be treated; an example of final dilution rate is 1:10. If a preformulate was used, the missing monobasic alkaline salt can also be conveniently added at this stage, for example dissolved in the diluting water.

For purposes of particular efficacy, it is preferable that in the solution as finally administered to the substrate: (a) the monobasic alkaline salt is present in a concentration comprised between 0.01 and 0.1 M, more preferably between 0.03 and 0.08 M, e.g. 0.07 M; (b) the pH of this solution is comprised between 4 and 6, more preferably between 4.4 and 5.7; within the ranges (a) and (b) the germination of conidia is particularly improved. Furthermore, in order to provide for an efficient degree of botrytization, the conidia concentration as finally administered is advisably comprised between 1 x 10⁶ and 6 x 10⁶ conidia /ml, more preferably between 2,2 x 10⁶ and 4,4 x 10⁶ conidia /ml, e.g. 3,1 x 10⁶ conidia/ml (c). The above values (a), (b), (c) can be present in the original composition or can be reached after proper dilution thereof from a more concentrate state. Therefore in one embodiment of the invention, the solutions/preformulates are provided to the user in a predetermined degree of concentration such that, upon performing a corresponding degree of dilution, they will provide composition ready to use falling within the above mentioned intervals (a), (b), (c).

When used for grapes botrytization, the present solutions are conveniently administered in amounts comprised between 3 and 7 ml / Kg grapes to be treated.

The general method of preparing the compositions of the invention includes the steps of adding to an aqueous medium:
a) the conidia of *Botrytis cinerea*
b) at least one C3-6 polyol
c) at least one monobasic alkaline salt.

Depending on convenience, the aqueous medium may be provided at once as a single aliquot to which the various ingredients are added; alternatively, it can be subdivided into one or more fractions to be added stepwise in the course of preparation, jointly with or separately from the various ingredients additions.

The steps a), b) c) may take place in any order, i.e. the aqueous medium may be supplemented in the order a-b-c, a-c-b, b-a-c, b-c-a, c-a-b, c-b-a. Preferably, the order of addition is a-b-c. According to a further embodiment, one or more among said steps may take place simultaneously: for example, the polyol and the salt may be added simultaneously. When a preformulate is produced, the process includes only steps a and b, whereas step c is performed separately prior to administration of the composition to the substrate.

The preparation of the composition conveniently includes a step of homogenisation: this can be attained by known methods like stirring, shaking, vibration, sonication (ultrasound treatment), etc.; sonication is especially preferred, being conveniently performed at a temperature between 5° and 10 °C, in particular between 6° and 10°C. Homogenisation can be performed at any times after the addition of *Botrytis* conidia; preferably it is performed immediately thereafter.

The composition of the invention can be provided to the user in a unified form, i.e. wherein *Botrytis cinerea* conidia, polyol and monobasic alkaline salt are contained together within a single volume of aqueous medium.

In another embodiment, the monobasic alkaline salt (in solid or dissolved form) is kept separate from the aqueous preformulate containing *Botrytis cinerea* conidia and polyol; the salt is preferably supplied in a form allowing easy mixing with the preformulate; such systems are well known in the art: they may e.g. consist in salt-filled pushable cap applied onto the preformulate container: upon application of pressure, the cap releases the salt into the preformulate; or the preformulate and the salt supply are part of a same container but are mutually separated by an easy removable/breakable barrier; alternatively the salt is formulated as a tablet, sachet, solution drops, or any other dosage unit which can be easily added to the preformulate; viceversa the liquid preformulate can be added into the salt supply container, etc. The invention includes a kit containing the preformulate and the salt supply in any forms suitable for mutual mixing.

The invention also includes a method to perform a controlled infection with *Botrytis cinerea* (botrytization) on a fruit substrate, in particular grapes. The method is characterised by contacting the substrate with a suitable amount of a composition as previously described. The methods of administering the composition can be chosen among those currently in use to treat fruit cultures with liquid means, i.e. spraying, watering, etc. Reference conditions (temperature, humidity, time) for incubation of the thus treated fruit are those commonly known (for grapes cf. e.g. C.S. Thomas, op.cit.). As a non-limitative reference, standard temperatures during the botrytization process are within 15-20°C, the relative humidity is between 65 and 100%, the treatment time is between 5 and 15 days, in a suitably ventilated environment.

However the use of the present compositions allows less stringent botrytization conditions, i.e. the latter can be varied to a significant extent without impact upon the quality of the treated product.

A further object of the invention is a method to prepare a botrytized wine, characterised by subjecting grapes or any related products (must, vinasse, etc.) to the traditional fermentation, wherein these substrates were previously subjected to botrytization by means of the present compositions. Normal fermentation conditions, as used for the production of traditional botrytized wines can be applied. The wine thus obtained, is preferably a sweet wine or dessert wine. They are characterised by a special quality, without presence of micotoxins originated by undesired side infections: Ocratoxins (below 0.1 ppb) and Aflatoxins (below 0.25 ppb), all being quite below the standard level admitted in wines.

In a variant, prior to fermentation the botrytized grape product can also be mixed with other varieties of grapes, musts, etc. (botrytized or not), obtaining a variety of wines where the sweet-aromatic component will affect the end taste in variable grades.

The invention achieves a number of industrially relevant advantages. The composition is a easy-to-handle, ready-to-use source of *Botrytis cinerea* suitable for botrytization processes. The present compositions remove the need to rely onto the naturally occurring *Botrytis* infection, the degree of which is very variable and/or is strictly reliant on climatic conditions. With respect to the known extemporaneously made *Botrytis* suspensions, it avoids the need of maintaining a lively culture of *Botrytis cinerea* and the uncertainty related to the size of the inoculus. The invention makes available a highly stable product, conveniently storable for long time without loss of efficacy. The constant titre of the fungus allows performing a desired level of controlled infection with a minor influence by climatic conditions; this allows in turn to obtaining high quality sweet wines, also from areas different from the traditional ones. The production in the traditional areas is also advantageously affected by the invention in that it is made more resistant against climatic changes (occurring more and more frequently in recent years). The production yield of sweet wines can thus be increased in all production sites. The more uniform and reproducible degree of infection allows a less stringent selection of the botrytized grapes to subjected to the subsequent fermentation step. It also allows the wine producer to better control/modify/adapt the infection process, either for the production of known wines or in the research for new varieties of sweet wines. Also the quality of the wine products is positively affected. In fact, the more intense and uniform infection obtained, reduces the risk of unwanted side infections from foreign microorganisms, to the advantage of final wine quality and product safety. This further effect (measured as a further reduction of normally accepted levels of unwanted mycotoxins) was experimentally ascertained by the present inventors in wines obtained from the present compositions.

The invention is now described by means of the following non-limiting examples.

### Experimental part

### Example 1: Preparation of a composition in sterile demineralised water, containing glycerol and conidia of Botrytis cinerea obtained from a culture on Potato Dextrose Agar.

The conidia are obtained from a monoconidial (i.e. derived from a single parent of *Botrytis cinerea*) culture of *Botrytis cinerea,* grown on a commercially available Potato Dextrose Agar.

The growing medium is prepared by mixing 39 g Potato Dextrose Agar (dehydrated powder) with 1000 ml demineralised water. The suspension is thus sterilised in autoclave for 15 minutes at 121°C. Thereafter the medium is maintained in a thermostated environment at 45°C ± 2°C. 300 ± 1000 ml of the medium are then transferred into aluminium trays with capacity of 2000 ml, suitable for fungal growth. All used materials have standard sterility conditions (sterility security level [SAL] < 10⁻⁶); these can be obtained e.g. by applying the sterilisation procedures mentioned above for the growing medium.

The *Botrytis cinerea* inoculum is prepared apart by inoculating a monoconidial culture of this microorganism (obtained from an isolate of the same) into 9 cm diameter Petri dishes containing potato dextrose agar; the cultures are incubated 18-20 days at 23- 27°C.

Portions of the thus obtained inoculum (each with a surface of 15.9 cm²) are placed, uniformly and under sterile conditions, onto the solidified medium previously prepared. The trays are then closed to further guarantee sterility and inner humidity of 80-90%, thereby favouring a correct growth of *Botrytis cinerea*; the trays are then incubated 18-20 days at 23-27°C. The fungal mycelium grows onto the agar medium surface, up to form a mycelium layer of 0.5-3mm. The fungal material (mainly conidia, with parts of mycelium) is collected in powdery form by recovering the medium surface up to a thickness of about 90% of the mycelium layer.

The thus collected conidia are quantified by direct microscope counting in a Thoma chamber: this is a special cell allowing the assessment of conidia within a given volume of sample (cf. Lebensm. Unters.Forsch., 1950, 91(2), 93-100); the datum is then expressed as number of conidia per gram of powder. 91 g of powder is then dispersed under stirring into 300 ml of sterile demineralised water. The suspension is then put into a refrigerated bath until reaching a temperature of 8° ± 2°C and then ultrasound-treated (sonication) by means of an immersion sonicator (three 30 sec. cycles, with potency within 100-150 watt). Thereafter the suspension is added with 100 g glycerol and brought to 1000 ml volume with sterile demineralised water. The thus obtained composition shows a storage stability of at least 1 year at temperatures between -15 and - 25 °C, or at least 30 days at temperatures between 3 and 5 °C. This composition is added with a 9 litre solution obtained by dissolving 9.1 g potassium monobasic phosphate per litre of demineralised water.

The preformulate germinability (germination potency) of the thus obtained solution is determined as follows: 30 µl of the solution are withdrawn and set on a microscope sample holder. The holder is placed into a cell with 100% relative humidity and incubated 24 hrs at 21±1°C. After incubation, the sample is covered with a glass and observed with an optical microscope, counting the number of germinated conidia per 100 observed conidia. The germination potency was found to be higher than 64%. Substantially identical potency was obtained from an identical composition which had undergone storing at -20°C for 1 year.

### Example 2: Preparation of a composition in sterile physiological water, containing glycerol and conidia of Botrytis cinerea obtained from a culture on Potato Dextrose Agar.

A compositions was prepared using the same procedures and quantities described in Example 1, but using sterile physiological water instead of sterile demineralised water. The germination potency, measured as per example 1, was found to be higher than 65%.

### Example 3: Preparation of a composition in sterile demineralised water, containing glycerol and conidia of Botrytis cinerea obtained from a culture on Sabouraud Dextrose Agar.

A compositions was prepared using the same procedures and quantities described in Example 1, but using as growing medium Sabouraud Dextrose Agar instead of Potato Dextrose Agar. The germination potency, measured as per example 1, was found to be higher than 70%.

### Example 4: Preparation of a composition in sterile demineralized water, containing mannitol and conidia of Botrytis cinerea obtained from a culture on Potato Dextrose Agar.

A composition was prepared using the same procedures and quantities described in Example 1, but using 100 g mannitol instead of 100 g glycerol. The germination potency, measured as per example 1, was found to be higher than 68%.

### Example 5: Preparation of a composition in sterile demineralized water, containing xylitol and conidia of Botrytis cinerea obtained from a culture on Potato Dextrose Agar.

A composition was prepared using the same procedures and quantities described in Example 1, but using 105 g xylitol instead of 100 g glycerol. The germination potency, measured as per example 1, was found to be higher than 82%.

### Example 6: Preparation of a composition in sterile demineralized water, containing sorbitol and conidia of Botrytis cinerea obtained from a culture on Potato Dextrose Agar.

A composition was prepared using the same procedures and quantities described in Example 1, but using 110 g sorbitol instead of 100 g glycerol. The germination potency, measured as per example 1, was found to be higher than 74%.

### Example 7: Preparation of a composition in sterile demineralized water, containing mannitol, glycerol and conidia of Botrytis cinerea obtained from a culture on Potato Dextrose Agar.

A composition was prepared using the same procedures and quantities described in Example 1, but using 50 g mannitol and 50 g glycerol instead of 100g glycerol. The germination potency, measured as per example 1, was found to be higher than 75%.

### Example 8: Preparation of a composition in sterile demineralized water, containing, glycerol, potassium monobasic phosphate and conidia of Botrytis cinerea obtained from a culture on Potato Dextrose Agar.

A composition was prepared using the same procedures and quantities described in Example 1, but using 91 g potassium monobasic phosphate and 100 g glycerol instead of 100 g glycerol. This composition is added with 9 litres of demineralised water instead of 9 litres solution obtained by dissolving 9.1 g potassium monobasic phosphate per litres of demineralised water. The germination potency, measured as per example 1, was found to be higher than 67%.

### Example 9: Preparation of a composition in sterile demineralized water, containing, mannitol, potassium monobasic phosphate and conidia of Botrytis cinerea obtained from a culture on Potato Dextrose Agar.

A composition was prepared using the same procedures and quantities described in Example 8, but using 100 g mannitol instead of 100 g glycerol. The germination potency, measured as per example 1, was found to be higher than 72%.

### Example 10: Preparation of a composition in sterile demineralized water, containing, xylitol, potassium monobasic phosphate and conidia of Botrytis cinerea obtained from a culture on Potato Dextrose Agar.

A composition was prepared using the same procedures and quantities described in Example 8, but using 105 g xylitol instead of 100 g glycerol. The germination potency, measured as per example 1, was found to be higher than 84%.

### Example 11: Preparation of a composition in sterile demineralized water, containing, sorbitol, potassium monobasic phosphate and conidia of Botrytis cinerea obtained from a culture on Potato Dextrose Agar.

A composition was prepared using the same procedures and quantities described in Example 8, but using 110 g sorbitol instead of 100 g glycerol. The germination potency, measured as per example 1, was found to be higher than 76%.

### Example 12: Preparation of a composition in sterile demineralized water, containing, mannitol, glycerol, potassium monobasic phosphate and conidia of Botrytis cinerea obtained from a culture on Potato Dextrose Agar.

A composition was prepared using the same procedures and quantities described in Example 8, but using 50 g mannitol and 50 g glycerol instead of 100 g glycerol. The germination potency, measured as per example 1, was found to be higher than 69%.

### Example 13: Wine analysis.

In independent experiments, the conidia composition of example 1 was used to infect different varieties of grapes (Chardonnay, Malvasia, Trebbiano, Sauvignon). The botrytied grapes were thereafter subjected to fermentation with natural *Saccharomyces,* applying the fermentation conditions normally in use for the respective varieties. Product safety analysis was performed by assessing compliance of the obtained wines with the standard allowable levels of mycotoxins.

Ocratoxin A was measured via the official method OIV MA-F-AS315-10-OCHRAT 2005 ("*Dosage de l'ocratoxine A dans le vin après passage sur colonne d'immunoaffinité et CLHP avec détection fluorimétrique"* issued by OIV *Organisation Internationale de la Vigne et du Vin*). Aflatoxins B1, B2, G1, G2 were assessed using the official methods AOAC 994.08 1995 and AOAC 999.07 2003 (issued by AOAC *Association of Official Analytic Chemists).*

All tested wines showed Ocratoxin A level below 0.1 ppb. In addition the levels of Alfatoxins B1, B2, G1, G2 were found consistently below 0.25 ppb for all wines. All the measured values are significantly lower than the standard tolerability levels in wine, showing that high quality botrytized wines were obtained for all the tested grapes varieties.

## Claims

1. Aqueous composition comprising: conidia of *Botrytis cinerea,* at least one C3-6 polyol, and at least one monobasic alkaline salt.

2. Composition according to claim 1, wherein the C3-6 polyol is chosen among glycerol, mannitol, xylitol, sorbitol, and mixtures thereof.

3. Composition according to any of claims 1-2, wherein the monobasic alkaline salt is monobasic sodium phosphate or monobasic potassium phosphate.

4. Composition according to any of claims 1-3, wherein the C3-6 polyol is present at a concentration comprised between 50 and 300g/l.

5. Composition according to any of claims 1-4, containing conidia of *Botrytis cinerea* at a concentration comprised between 1 X 10⁶ and 6 X 10⁶ conidia /ml.

6. Composition according to any of claims 1-5, having pH comprised between 4 and 6 and wherein the monobasic alkaline salt is present in a concentration comprised between 0.01 and 0.1 M.

7. Composition according to any of claims 1-6 containing, as aqueous medium, demineralised water, distilled water, or physiological solution.

8. Kit comprising a preformulate aqueous composition comprising conidia of *Botrytis cinerea* and at least one C3-6 polyol, and a supply of at least one monobasic alkaline salt meant for admixture with said preformulate composition.

9. Kit according to claim 8, where the preformulate and the salt supply are present in amounts such that, after mutual admixture and possible final dilution, they provide a solution wherein the monobasic alkaline salt is present in a concentration comprised between 0.01 and 0.1 M and having pH comprised between 4 and 6.

10. Kit according to any of claims 8-9, where the preformulate and the salt supply are present in amounts such that, after mutual admixture and possible final dilution, they provide a solution wherein the conidia of *Botrytis cinerea* are present at a concentration comprised between 1 X 10⁶ and 6 X 10⁶ conidia /ml.

11. Method for botrytization of grapes or related products, comprising the step of contacting them with a composition according to any of claims 1-7.

12. Method for the production of botrytized wine, comprising the step of fermenting grapes or related products previously being infected with *Botrytis cinerea* by means of a composition according to any of claims 1-7.

## Patentansprüche

1. Wässrige Zusammensetzung umfassend: Konidien von *Botrytis cinerea,* mindestens ein C3-6 Polyol und mindestens ein einbasiges alkalisches Salz.

2. Zusammensetzung nach Anspruch 1, in dem das C3-6 Polyol ausgewählt ist aus Glycerin, Mannitol, Xylitol, Sorbitol, und Mischungen davon.

3. Komposition nach irgendeinem der Ansprüche 1 bis 2, in dem das einbasige alkalische Salz ein einbasiges Natriumphosphat oder einbasiges Kaliumphosphat ist.

4. Zusammensetzung nach irgendeinem der Ansprüche 1 bis 3, neben das C3-6 Polyol in einer Konzentration zwischen 50 und 300 g/l anwesend ist.

5. Komposition nach irgendeinem der Ansprüche 1 bis 4, enthaltend Konidien von *Botrytis cinerea* in einer Konzentration zwischen 1 x 10⁶ und 6 x 10⁶ Konidien pro ml.

6. Komposition nach irgendeinem der Ansprüche 1 bis 5, aufweisend einen pH zwischen 4 und 6 und wobei das einbasige alkalische Salz in einer Konzentration zwischen 0,01 und 0,1 M anwesend ist.

7. Komposition nach irgendeinem der Ansprüche 1 bis 6, beinhaltend als wässriges Medium, demineralisiertes Wasser, destilliertes Wasser, oder physiologische Lösung.

8. Set umfassend eine vorformulierte wässrige Zusammensetzung umfassend Konidien von *Botrytis cinerea* und mindestens ein C3-6 Polyol, und einen Vorrat von mindestens einem einbasigen alkalischen Salz gedacht zur Zumischung zu der vorformulierten Zusammensetzung.

9. Set nach Anspruch 8, in dem die Vorformulierung und der Salzvorrat vorhanden sind in solchen Mengen, dass nach der gegenseitigen Vermischung und möglichen Endverdünnung sie eine Lösung bereitstellen, in der das einbasige alkalische Salz in einer Konzentration zwischen 0,01 und 0,1 M vorhanden ist und einen pH zwischen 4 und 6 aufweist.

10. Set nach irgendeinem der Ansprüche 8 bis 9, in dem die Vorformulierung und der Salzvorrat vorhanden sind in solchen Mengen, dass nach gegenseitiger Vermischung und möglicher Endverdünnung sie eine Lösung bereitstellen, in der die Konidien von *Botrytis cinerea* in einer Konzentration zwischen 1 x 10⁶ und 6 x 10⁶ Konidien pro ml vorhanden sind.

11. Verfahren zur Botrytisation von Trauben oder verwandten Produkten, umfassend den Schritt deren Kontaktierung mit einer Zusammensetzung gemäß einem der Ansprüche 1 bis 7.

12. Verfahren zur Produktion von botrytisiertem Wein, umfassend den Schritt der Fermentation von Trauben oder verwandten Produkten die zuvor mit *Botrytis cinerea* infiziert worden waren mittels einer Zusammensetzung gemäß einem der Ansprüche 1 bis 7.

## Revendications

1. Composition aqueuse comprenant des conidies de *Botrytis cinerea,* au moins un polyol C3-6, et au moins un sel alcalin monobasique.

2. Composition selon la revendication 1 dans laquelle le polyol C3-6 est choisi parmi le groupe comprenant le glycérol, le mannitol, le xylitol, le sorbitol, et des mélanges de ceux-ci.

3. Composition selon l'une quelconque des revendications 1-2, dans laquelle le sel alcalin monobasique est le phosphate de sodium monobasique ou le phosphate de potassium monobasique.

4. Composition selon l'une quelconque des revendications 1 à 3, dans laquelle le polyol C3-6 est présent à une concentration comprise entre 50 et 300 g/l.

5. Composition selon l'une quelconque des revendications 1 à 4, contenant des conidies de *Botrytis cinerea* à une concentration comprise entre 1 X 10⁶ et 6 X 10⁶ conidies/ml.

6. Composition selon l'une quelconque des revendications 1 à 5, ayant un pH compris entre 4 et 6 et dans laquelle le sel alcalin monobasique est présent à une concentration comprise entre 0,01 et 0,1 M.

7. Composition selon l'une quelconque des revendications 1 à 6 contenant comme milieu aqueux de l'eau déminéralisée, de l'eau distillée, ou une solution physiologique.

8. Kit comprenant une composition aqueuse pré-formulée comprenant des conidies de *Botrytis cinerea* et au moins un polyol C3-6, et un supplément d'au moins un sel alcalin monobasique utilisable pour mélange avec ladite composition pré-formulée.

9. Kit selon la revendication 8, où la composition pré-formulée et le supplément de sel sont présents en quantité telles que, après leur mélange et une possible dilution finale, on obtienne une solution dans laquelle le sel alcalin monobasique est présent dans une concentration comprise entre 0,01 et 0,1 M et ayant un pH compris entre 4 et 6.

10. Kit selon la revendication 8 ou 9, où la composition pré-formulée et le supplément de sel sont présents dans des quantités telles que, après leur mélange et une possible dilution finale, on obtienne une solution dans laquelle les conidies de *Botrytis cinerea* sont présentes dans une concentration comprise entre 1 X 10⁶ et 6 X 10⁶ conidies/ml.

11. Méthode de botrytisation du raisin ou des produits apparentés, comprenant une étape les mettant en contact avec une composition selon l'une quelconque des revendications 1 à 7.

12. Méthode de production de vin botrytisé, comprenant une étape de fermentation du raisin ou de produits apparentés ayant précédemment été infectés par le *Botrytis cinerea* grâce à une composition selon l'une quelconque des revendications 1 à 7.
